# EUROPEAN PATENT APPLICATION

(11) **EP 1 508 386 A1**
(43) Date of publication of application: **23.02.2005**
(21) Application number: 04018785.8
(22) Date of filing: 09.08.2004
(51) Int. Cl.: B09B 3/00, C12M 1/107, F23G 5/44

(54) **Method for waste treatment**

(30) Priority: 13.08.2003 FI 20031151
(71) Applicant: Lassila & Tikanoja Oyj, 00441 Helsinki (FI)
(72) Inventor: Hietanen, Lassi, 40900 Säynätsalo (FI)
(74) Representative: Laitinen, Pauli Sakari

(57) **Abstract**

A method for waste treatment with the purpose of utilising the waste. The method comprises the following steps:
- baling collected waste (1);
- activating waste closed in bales for accelerating anaerobic bacterial production and through that the starting and continuation of generation of gas;
- locating bales in an essentially gas-tight reactor (2) in which they are kept as long as essentially all generation of gas has finished;
- recovering gas generated in the reactor for recycling (3); and
- disposing the resulted quantity of waste decreased in volume to a desired final disposal site (6).

## Description

This invention relates to waste treatment and, more closely, to a method with which it is possible to utilise the valuable parts of waste and to reduce the quantity of waste.

In modern society, enormous quantities of waste are produced. Most of waste ends up in landfills, the tight environmental standards relating to which will increase costs a great deal after year 2007. In addition, there will be absolute prohibitions for taking some waste to landfills. Certain utilisable waste fractions cannot be taken to a landfill at all. One way is to recover materials utilised in secondary use, such as glass and paper. Likewise, by composting biological material, one tries to have biological material for recycling.

A general increase in packaging etc. material is, however, more and more a problem because of changed lifestyles. In addition, certain utilisation methods, such as incinerating, are restricted by strict standards regarding emissions of combustion gases. Unless one will be able to proceed considerably further in its sorting, waste includes so many harmful materials that incinerating is not a rational choice. Even though emissions may be managed, a technical problem exists regarding incinerating low-quality waste: it gives only small quantities of electric power. Most of the gained energy is heat.

Landfill standards are, as mentioned above, quite strict, and new, even stricter directives of the following few years have especially caused significant changes in landfills. A way of partial utilisation of landfills is to recover landfill gases, the main component of which is methane, which as a combustible gas may be utilised by burning and by recovering the heat generated by the burning to be used, for example, for district heating requirements. However, it requires that there is a suitable community using district heating in the vicinity of the landfill. This is not always the case, or the community receives the heat it requires as the lost heat of a local power plant generating electric power and heat, that is, combined heat and power (CHP) generation is used. Landfill gas may also be used as engine fuel.

A landfill generates gas for many decades. It is evident that the output on such a long period is not very large. This creates problems regarding the recovery of gas and its use. However, when the generation of gas has ended in due time, despite having been recovered or not, the quantity of waste has approximately halved, which means considerable savings in terms of the end-storing volume of waste.

The object of this invention is to achieve a method by means of which one may, in a rational way, recover a part of the landfill waste volume with a relatively fast schedule, after which the smaller end-volume of landfill waste may be disposed of finally.

As waste includes many such components which create a risk for employers in the field, waste should be treated so that no danger is caused for employers or the environment. In a small scale, waste has been baled, whereby a waste batch wrapped in plastic is clearly less risky to its environment. Baling of waste is also an aspect of this invention.

The invention is next described by referring to a simplified diagrammatical figure which provides the general terms of the method steps according to the invention.

Thus, the first step in the method according to the invention is the baling of waste. This is illustrated in the enclosed diagram by a baling station 1. It is evident that baling may possibly be performed also in another way than bringing the waste to a special baling station. One may consider, for example, that baling is included in the features of a waste collecting truck, in which case the waste entering the following step would be already wrapped when unloaded from the truck.

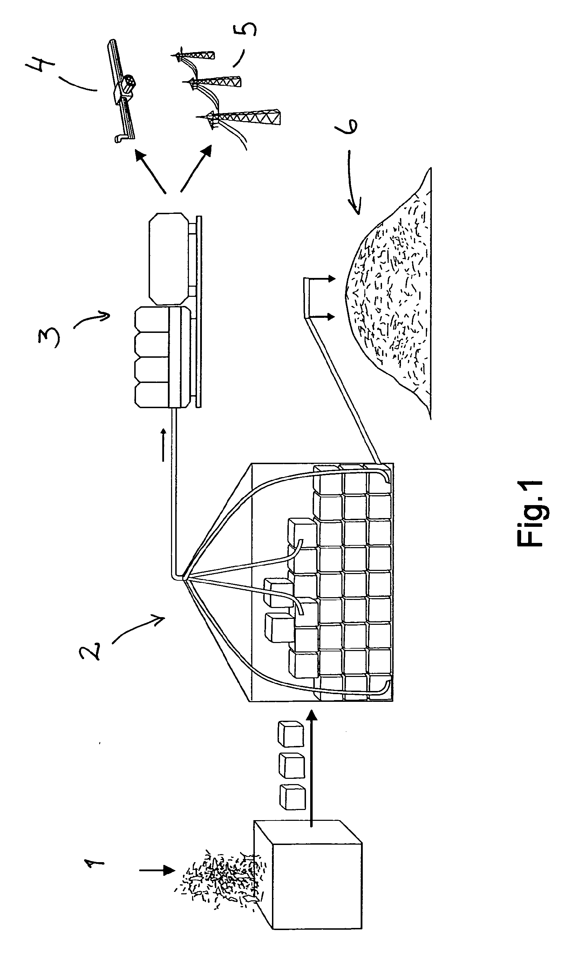

The waste wrapped according to the invention is piled compactly to a tight-walled reactor 2 which is also covered closely and is equally equipped with gas-collecting pipes, which are not drawn to the figure for clarity. After this, one awaits for the gas formation to start, after which the gas is recovered and utilised in a suitable way. A suitable way may be, for example, a diesel power station which is illustrated in the figure by reference number 3. This kind of power plant generates, at its best, electric power 5 and also heat 4. Alternatively, gas may simply be burned to generate heat.

An essential feature of the method according to the invention is that the generation of gas is accelerated so that the gas formation time, counted in prior-art methods in dozens of years, has been decreased by means of acceleration to a few years, for example, to 2-3 years. It is usually enough for achieving acceleration that an adequate quantity of moisture is brought to waste bales. This may be accomplished, for example, by spraying them with water or with water including certain nutrients. Thus, the anaerobic process accelerates considerably, and the generation of gas starts more rapidly than in the conventional way, gas is generated more intensely, and all generation of gas finishes quicker. From this follows that the reactor batch may be replaced with a new one, and the process may be started from the beginning. With the process, the quantity of waste has decreased, and at the same time, its biological activity has exhausted, and thus one has saved space required by end-storing, and fulfilled the requirement that biological activity no longer exists in the landfill.

As mentioned above, burning generated gas and thus generating heat especially for district heating requirements is a simple and easy way to utilise gas. This requires, however, that there is an adequate number of heat consumers within sensible distance. Because it is not likely that people choose to have waste disposal plants very close to residential areas, although it would be conceivable utilising the present invention because of its cleanliness, the lack of an adequate quantity of district heating load becomes a problem regarding burning. The waste incineration with a conventional technique requires a quantity of 100,000 tons of waste, and it will create 300-500GWh of heat which corresponds to the district heating network heat requirement of a town of 30,000-50,000 inhabitants.

By utilising, in the method functioning according to the invention, a diesel power station and the generated gas as its fuel, one achieves fairly good efficiency compared to any current plant intended for utilisation of landfills, and one is able to operate in essentially smaller units. Heat may be generated according to requirement, but the utilisation is mainly gained as electric power which is easy to supply to the network for any kind of end-use.

The invention provides a clear improvement compared to current systems, because it enables the utilisation of mixed waste of lowest quality, and the gained utility is received as the most valuable form of energy, that is, electric power. The waste problem as such will not be solved in this way, but this is a step forward applied to the current system. Mixed waste includes typically around 1% of chlorine, 30% of ashes, 35% of moisture and a lot of heavy metals and impurities. Now the incineration of this extremely problematic fuel may be avoided, and the energy source, that is, carbon is recovered biologically as methane so that the impurities remain in the final disposal.

## Claims

1. A method for waste treatment with the purpose of utilising the waste, **characterised in that** it comprises the following steps:
- baling collected waste (1);
- activating waste closed in bales for accelerating anaerobic bacterial production and through that the starting and continuation of generation of gas;
- locating bales in an essentially gas-tight reactor (2) in which they are kept as long as essentially all generation of gas has finished;
- recovering gas generated in the reactor for recycling (3); and
- disposing the resulted quantity of waste decreased in volume to a desired final disposal site (6).

2. A method according to Claim 1, **characterised in** performing the activation of baled waste by adding moisture inside a bale by, for example, spraying the bale with water.

3. A method according the Claim 1, **characterised in** locating a pipe system to the reactor (2) for recovering gas.

4. A method according to any one of above claims, **characterised in** burning the generated and recovered gas or utilising it as a fuel in an engine-driven power unit.

5. A method according to Claim 4, **characterised in** using the generated gas as fuel in a diesel power station producing electric power.

6. A method according to any one of above claims, **characterised in** baling waste in bales wrapped in plastic.
